# EUROPEAN PATENT APPLICATION

(11) **EP 2 044 931 A2**
(43) Date of publication of application: **08.04.2009**
(21) Application number: 08165800.7
(22) Date of filing: 03.10.2008
(51) Int. Cl.: A61K 9/14, A61P 29/00

(54) **Pharmaceutical compositions based on porous zeolites as release means of pharmacologically active molecules and relative use**

(30) Priority: 05.10.2007 IT MI20071930
(71) Applicant: SASOL ITALY S.p.A., 20124 Milan (IT)
(72) Inventor: Rimoli, Maria Grazia, 80014, GIUGLIANO (Napoli) (IT); Rabaioli, Maria Roberta, 21052, BUSTO ARSIZIO (Varese) (IT); Abignente, Enrico, 80127, NAPOLI (IT); Melisi, Daniela, 74100, TARANTO (IT); Mirabelli, Rosella, 88821, ROCCA DI NETO (Crotone) (IT); Novellino, Ettore, 83040, MONTEMARANO (Avellino) (IT); Curcio, Annalisa, 80058, TORRE ANNUNZIATA (Napoli) (IT); De Lucia, Salvatore, 82011, FORCHIA (Benevento) (IT); Nasti, Alessandro, 80126, NAPOLI (IT)
(74) Representative: De Gregori, Antonella

(57) **Abstract**

The present invention relates to pharmaceutical compositions for the oral administration of pharmacologically active molecules, comprising a release means consisting of porous zeolite in powder form, incorporating pharmacologically active molecules inside the pores and/or on its surface, the relative use for the preparation of drugs for oral administration, in particular for the treatment of inflammatory pathologies at an intestinal level and the preparation process of these compositions.

## Description

The present invention relates to pharmaceutical compositions based on porous zeolites as release means of pharmacologically active molecules and the relative use thereof.

The present invention falls within the technical field of release means of pharmacologically active molecules, i.e. of means or carriers for carrying pharmacologically active molecules inside the human body and/or mammals. In particular, the present invention relates to a means or carrier for the oral administration of non-steroidal anti-inflammatory drugs (FANS), said means being capable of carrying the pharmacologically active molecules of the above drugs as far as the absorption sites of the intestine, protecting them from the deactivating action of substances naturally present in the stomach.

Pathologies in human beings and mammals are commonly treated with drugs containing active principles which are administered orally to the subject to be treated in the form of tablets, capsules or pills. Oral administration is in fact the simplest form of taking a drug, in addition to being safe and economical with respect to other types of administration. Some of the drugs generally administered orally (for example FANS), however, contain active principles which can be attacked by gastric juices and digestion enzymes and consequently remain ineffective. In other cases, on the other hand, the molecules of the active principles are unfavorably influenced by the high acidity conditions which characterize the stomach, which can induce chemical modification processes of the molecules with a consequent loss in the original pharmacological efficacy.

In all these cases, in order to guarantee a sufficient bioavailability of the active principle, i.e. to guarantee that the pharmacologically active molecules of the drugs reach the absorption sites of the intestine in a sufficiently high quantity to exert an effective pharmacological action, thus compensating the loss in efficacy which the active principle can undergo during its passage through the stomach, it is necessary to increase the dose of the drug to be administered. High dosages of drugs, however, are not desirable due to the side-effects which can arise in the organism.

A second problem associated with the oral administration of drugs is linked to the possible undesired effects which some active principles have on a gastro-intestinal level and, in particular, at the level of the stomach. The administration of ketoprofene (2-(3-benzoylphenyl)propanoic acid, however, one of the FANS most commonly used in the treatment of intestinal inflammatory pathologies (such as for example ulcerative colitis, Crohn's disease), is frequently accompanied by disturbances to the stomach, in some cases also quite serious (for example hemorrhages).

In order to avoid these problems, various types of means or carriers suitable for oral administration, suitable for protecting the pharmacologically active molecules in their passage towards the intestine, have been developed. These means generally consist of casings (for example gelatin capsules) containing the pharmacologically active molecules in their interior, said casings being capable of at least partially resisting the attack of juices and/or gastric enzymes.

Once these means or carriers have reached the intestine, they release molecules of active principle which can therefore be absorbed by the organism and exert their pharmacological action.

This type of means or carrier, however, has the disadvantage that the patient not only takes the drug but also the other substances that form the casing with possible side-effects due, for example, to intolerance or hypersensitivity to these substances.

New release means or carriers for the oral administration of drugs have recently been developed, essentially consisting of porous zeolites which incorporate molecules of active principle.

The zeolites, of both a natural and synthetic origin, are aluminosilicates of a porous crystalline nature consisting of a three-dimensional framework of TO₄ tetrahedra, wherein T can be a silicon or aluminum atom bound in tetrahedral coordination to 4 oxygen atoms. The presence of aluminum in tetrahedral coordination in the crystalline lattice causes an excess negative charge which is balanced by metallic ions, generally ions of alkaline metals (Na, K, Rb, Li and Cs), alkaline earth metals (Mg and Ca) and ammonium cations. The zeolites also contain water molecules, weakly bound, which can be easily removed by heating in order to release a wide surface area and make the pore volume accessible.

In pharmaceutical compositions containing zeolites, the zeolites act as "carriers" of pharmacologically active molecules, transporting them as unaltered as possible to the absorption sites where they exert their action.

The use of zeolites as means or carriers for transporting and administering or releasing active principles is known in the preparation of pharmaceutical compositions for the treatment of pathologies of the skin. WO 02/100420, for example, describes the use of a natural zeolite (clinoptilolite), containing zinc and erythromycin, for the treatment of acne. The technical problem which the above patent application attempts to solve is delaying or preventing the growth of bacterial strains resistant to the antibiotic erythromycin thanks to the combined release of Zn with the molecules of the antibiotic. This patent application exclusively describes the use of zeolites for the preparation of pharmaceutical compositions for topical application.

The necessity is felt in the state of the art for identifying new release means or carriers for the oral administration of pharmacologically active molecules, said means being capable of carrying these molecules as far as the absorption sites situated in the intestine of human beings and/or other mammals, protecting them from the activity of the juices and gastric enzymes of the stomach and/or without causing undesired side-effects.

The objective of the present invention is to find a release means or carrier for the oral administration of pharmacologically active molecules capable of overcoming the drawbacks described of the known art.

An object of the present invention therefore relates to a pharmaceutical composition for the oral administration of pharmacologically active molecules, comprising a release means consisting of porous zeolite in powder form incorporating pharmacologically active molecules.

An object of the present invention also relates to the use of the pharmacological composition comprising a release means consisting of a porous zeolite in powder form incorporating pharmacologically active molecules inside the pores and/or on its surface, for the preparation of drugs for oral administration, in particular for the treatment of inflammatory intestinal pathologies.

A further object of the present invention relates to the release means for the oral administration of pharmacologically active molecules consisting of a porous zeolite in powder form selected from zeolites belonging to the group of faujasites (type Y, X, ALSX) or to the group of A-type zeolites (LTA), even more preferably X-type zeolites and/or ALSX.

Another object of the present invention relates to the process for the preparation of a pharmaceutical composition for the oral administration of pharmacologically active molecules, comprising a release means consisting of porous zeolite in powder form incorporating pharmacologically active molecules inside the pores and/or on its surface, said process comprising the following operational steps:
a) heating the porous zeolite in powder form to a temperature within the range of 200 to 600°C, for a time varying from 0.5 to 16 hours;
b) putting the zeolite obtained from phase a) in contact, at a temperature ranging from 25 to 30°C for a time ranging from 36 to 48 hours, with a charging solution comprising pharmacologically active molecules dissolved in a solvent or a mixture of solvents;
c) separating the zeolite from the solvent.

In particular, according to the present invention, the porous zeolite in powder form incorporates the pharmacologically active molecules inside the pores and/or on its surface.

The term "incorporating" means that the zeolite is charged with pharmacologically active molecule inside the pores and/or on its surface, preferably both inside the pores and on its surface.

According to the present invention, the porous zeolites which can be used as release carriers or means of pharmacologically active molecules are generally those having pore dimensions which are sufficiently large as to house FANS molecules. The zeolites which can be used as carriers are preferably selected from zeolites belonging to the groups of faujasites (type Y, X, ALSX) or to the group of A-type zeolites (LTA), even more preferably X-type zeolites and/or ALSX.

Zeolite X is a zeolite belonging to the group of faujasites, characterized by an Si/Al ratio in the framework within the range of 1 ≤ Si/Al ≤ 1.5 and a non-uniform pore system (areas with pores having an average diameter equal to 7.4 Å and areas with pores having an average diameter equal to 2.2 Å).

Zeolite ALSX is an aluminosilicate obtained according to a co-crystallization procedure of zeolite X and zeolite A. The synthesis procedure is described, for example, in a previous patent of the Applicant (IT 1284078). Zeolite ALSX has an Si/Al ratio similar to that of zeolite A, within the range of 1.05-1.3 extremes included, and the same crystalline structure as zeolite X. It also has a high ion-exchange capacity and rate, in particular of calcium and magnesium ions, much higher than those which can be obtained, for example, by simple mechanical mixing of type A zeolites and type X zeolites. Zeolite ALSX has a porous structure consisting of a system of three-dimensional channels with pores having a non-uniform width similar to those of zeolite X. In addition to these characteristics, zeolite ALSX has an extremely high surface area due to its particular physical structure characterized by closely bound co-crystals of zeolite A and zeolite X.

For the preparation of the pharmaceutical compositions according to the present invention, zeolites containing various types of balancing cations of the negative charge of the framework, can be used as release carrier or means. Zeolites are preferably used in which the balancing cations are Na⁺ and/or K⁺, even more preferably zeolites in sodium form in which the balancing cation is Na⁺. Zeolites with other cations of alkaline, alkaline earth metals, H⁺, NH₄⁺, can also be used, however.

In order to use the above zeolites as release means or carriers in the pharmaceutical compositions according to the present invention, it is necessary to charge the pharmacologically active molecules inside the pores and/or on the surface. For this purpose, before charging, the zeolites must be subjected to phase a) of the process according to the present invention, i.e. to an activation treatment comprising heating the zeolite to a temperature ranging from 200 to 600°C, to remove the molecules of water normally adsorbed inside the pores and on the surface of the crystals.

The heating is preferably prolonged for a time varying from 3 to 4 hours, to a temperature ranging from 300 to 400°C, even more preferably to a temperature of 350°C. For example, the zeolites of the X and ALSX type used for preparing the pharmaceutical compositions according to the present invention, were activated with heating in an oven to 350°C for about 4 hours.

The Applicant has observed that the activation of the zeolite is an indispensable operation for the preparation of effective release means for the purposes of the present invention. Without this activation, in fact, the subsequent charging operation of the pharmacologically active molecules is ineffective as shown in the examples. The water molecules adsorbed on the zeolite prevent access to the pores on the part of the pharmacologically active molecules.

The charging of the zeolite with the pharmacologically active molecules is effected by impregnation of the zeolite with liquid solutions of these molecules in one or more solvents (charging solutions) and subsequent evaporation of the solvent and/or solvents.

The impregnation is effected by dispersing, at a temperature within a range varying from 25 to 30°C, the activated zeolite in a charging solution and keeping the dispersion under stirring for a time ranging from 36 to 48 hours. The zeolite impregnated with the pharmacologically active molecules is subsequently separated by filtration from the solvent or mixture of solvents. Once separated, the zeolite impregnated with the pharmacologically active molecules is further dried, under vacuum, in a rotating evaporator (Rotavapor).

In a preferred embodiment of the present invention, the pharmacologically active molecules which can be incorporated in the release means consisting of zeolites, in particular type X and ALSX, are molecules of a non-steroidal anti-inflammatory drug (FANS).

The pharmacologically active molecules are preferably molecules selected from the group of derivatives of acetic acid (for example indomethacin, diclofenac, ketorolac, etc.), derivatives of propionic acid (for example ibuprofene, ketoprofene, naproxen, flurbiprofene, etc.), fenamate derivatives (for example mefenamic acid, meclofenamate, etc.), selective FANS Cox-2 (for example rofecoxib, celecoxib, etoricoxib, etc.) oxicams (for example, pyroxicam, meloxicam, tenoxicam, lornoxicam, cinnoxicam), nimesulide and/or mixtures thereof.

More preferably, the molecules are selected from indomethacin, diclofenac, ketorolac, ibuprofene, ketoprofene, naproxen, flurbiprofene, mefenamic acid, meclofenamate, rofecoxib, celecoxib, etoricoxib, pyroxicam, meloxicam, tenoxicam, lornoxicam, cinnoxicam, nimesulide and/or mixtures thereof, and even more preferably they are molecules of ketoprofene.

In a preferred embodiment of the pharmaceutical composition according to the present invention, the pharmaceutical composition for the oral administration of pharmacologically active molecules, comprises a release means consisting of porous zeolite in powder form of the X and ALSX type, incorporating pharmacologically active molecules of ketoprofene inside the pores and/or on its surface.

The charging solutions for incorporating FANS molecules in the zeolites are prepared by dissolving the FANS molecules in a solvent selected from the group comprising ethyl ether, ethyl alcohol, acetone and/or mixtures thereof. The solvent used is preferably ethyl ether.

The charging solutions can be prepared either starting directly from the FANS molecules or starting from the respective sodium salts. In the case of charging solutions prepared starting from sodium salts it is preferable to use ethyl alcohol as solvent.

It has been observed that the quantity of FANS molecules which can be charged in the zeolites varies in relation to the type of solution used. In particular, the charging levels of the FANS in the zeolites, impregnated with charging solutions prepared starting from the corresponding sodium salts, are extremely low (typically, lower than 3% by weight of the FANS with respect to the total weight of the activated zeolite subjected to impregnation). A possible reason for the low charging levels observed could be the repulsive interaction between the negative charges of the zeolite lattice and those of the anions of the sodium salt of the FANS.

The charging solutions can have a varying concentration of the FANS to be incorporated in the pores of the zeolite and/or on its surface, said concentration varying in relation to the charging degree to be obtained. The concentration of FANS of a charging solution typically varies from 10 g/l to 50 g/l, preferably from 20 to 30 g/l.

The pharmaceutical compositions according to the present invention were subjected to tests in vitro to evaluate the effectiveness of the release means or carrier in releasing pharmacologically active molecules of FANS in relation to the pH conditions in the environment in which they are acting. For this purpose, the pharmaceutical compositions comprising X and ALSX zeolites charged with ketoprofene were dispersed in aqueous solutions at certain pH values. The pH values selected, together with the ionic composition of the aqueous solutions, intend to simulate the conditions present in the gastro-intestinal system of human beings and, in general, of mammals.

The pharmaceutical compositions based on X and ALSX type zeolites proved to be extremely effective in releasing molecules of ketoprofene under specific conditions of acidity. In particular, the above release means showed a complete release of the molecules of ketoprofene for pH values higher than 6.5, whereas for pH values around 1 the release proved to be very limited (less than 10% by weight of the overall quantity of ketoprofene incorporated in the zeolite).

The results of the tests in vitro lead to the conclusion that the pharmaceutical compositions according to the present invention, based on porous zeolites as release means of pharmacologically active molecules, by not releasing FANS molecules in solutions with low pH values, are capable of protecting these molecules during their passage through the stomach and transporting them unaltered as far as the intestine, where they are released and absorbed by the organism.

Although the release mechanism of the pharmacologically active molecules has not yet been fully understood, it can be assumed that the pH value influences the equilibrium between the protonated form and deprotonated form (anionic) of the ketoprofene molecules, shifting them towards the latter, with a consequent repulsion on the part of the zeolitic framework and release into the surrounding environment.

The release capacity of FANS on the part of the release means according to the present invention can be favourably exploited for the preparation of pharmaceutical compositions containing these means. In particular, the above release means are used for the preparation of pharmaceutical compositions for the treatment of inflammatory pathologies, such as for example ulcerative colitis and Crohn's disease.

There are numerous advantages of the pharmaceutical compositions based on porous zeolites as release means according to the present invention. Firstly, the zeolite allows the pharmacologically active molecules to be carried directly to the absorption sites in the intestine, increasing the bioavailability in the action sites and thus allowing a reduction in the doses of drug to be administered, with the same pharmacological effectiveness.

A second advantage is linked to the possibility of releasing the FANS molecules in a specific way and in a controlled quantity, which allows undesired interactions between the drugs and parts of the gastro-intestinal system not interested in the pharmacological action, to be eliminated or at least reduced. In the case of the administration of ketoprofene, for example, troublesome stomach disturbances, which accompany the administration of this drug with the release means of the pharmaceutical compositions known in the state of the art, can thus be avoided.

Synthetic zeolites also have the advantage of being able to be prepared with a high degree of purity and with a well-defined chemical composition. This makes the release means according to the present invention particularly suitable for use in the preparation of pharmaceutical compositions to be administered orally.

The potentiality of these release means also lies in the possibility of being synthesized and/or selected in relation to the geometrical characteristics and steric hindrance of the pharmacologically active molecules to be incorporated inside the pores and/or on the surface of the zeolite.

The following embodiment examples are provided for purely illustrative purposes of the present invention and should not be considered as limiting the protection scope defined by the enclosed claims.

### Example 1: Characterization of the zeolites (release carriers or means)

For the preparation of the release carriers or means of the pharmaceutical compositions according to the present invention, two different starting zeolites were used, one of the X type (Sample A) and one of the ALSX type (Sample B).

The chemical composition of the starting zeolites was determined by means of elemental analysis with the ICP-AES technique. The compositions of the two zeolites are indicated in Table 1.

**Table 1 Chemical composition (weight % with respect to the total weight of the zeolite)**

| | **Na₂O** | **K₂O** | **Al₂O₃** | **SiO₂** | **H₂O** |
|---|---|---|---|---|---|
| Sample A | 14.6 | 0.0 | 24.0 | 35.4 | 26.0 |
| Sample B | 14.4 | 1.2 | 25.0 | 33.6 | 25.8 |

The structural analysis of the zeolites was effected by means of X-ray diffraction, which allowed the structural parameters indicated in Table 2 to be determined. The Si/Al ratio in each zeolite was calculated using the D.W. Breck equation.

**Table 2. X-ray Diffractometry (XRD)**

| | **Type of zeolite** | **Cell parameter (Å)** | **Si/Al** |
|---|---|---|---|
| Sample A | X | 24.940 | 1.23 |
| Sample B | ALSX | 25.007 | 1.04 |

The cell parameter indicated is that of zeolite X, from which the Si/Al ratio of zeolite X and X phase of zeolite ALSX, is obtained.

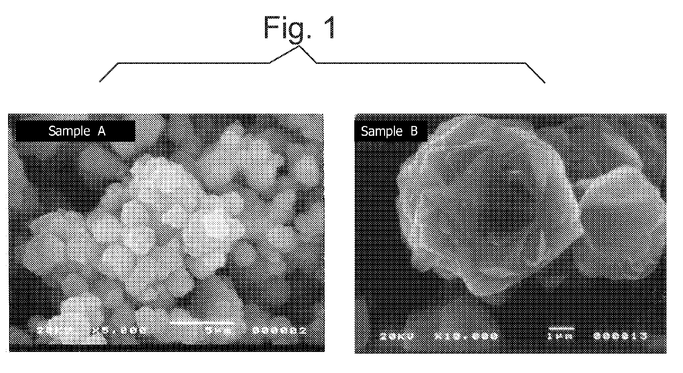

The purity and crystalline structure of the zeolites were confirmed by scanning electron microscopy (SEM). Two SEM images of samples A and B are indicated in Figure 1.

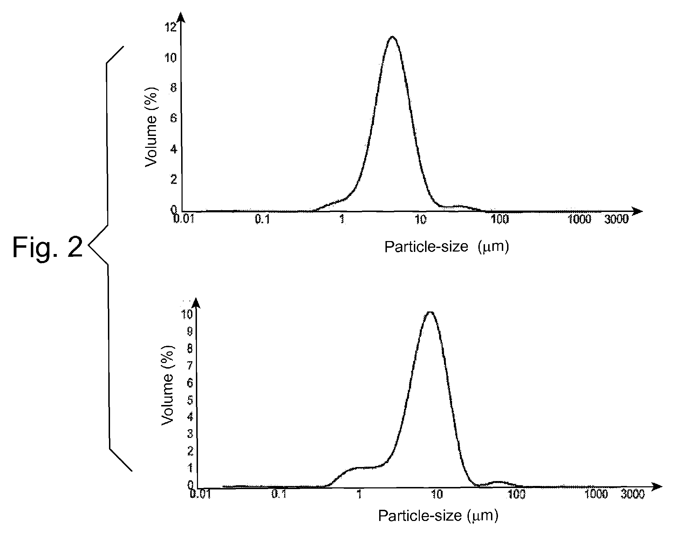

The particle-size distribution of the zeolites was determined by laser diffraction with a Malvern Mastesizer 2000 instrument. The results of the determination are indicated in Figure 2. In the case of zeolite X (Sample A), the average particle diameter proved to be about 7.5 µm, whereas for zeolite ALSX (Sample B) was about 4.5 µm.

### Example 2 - Charging of the FANS molecules

Seven different pharmaceutical compositions containing ketoprofene molecules were prepared starting from samples A and B of Table 2.

In compositions 1-4, the charging of the zeolite was effected with a charging solution containing 800 mg of ketoprofene dissolved in 30 ml of ethyl ether.

For the preparation of compositions 1 and 5, zeolite X (Sample A) and for the preparation of compositions 2 and 6, zeolite ALSX (Sample B), before the charging, were activated by heating in an oven to 350°C for 4 hours.

For the preparation of composition 3, zeolite X (Sample A) and for the preparation of compositions 4 and 7 zeolite ALSX (Sample B), on the other hand, were subjected directly to charging without any preliminary activation treatment.

In compositions 5-7, the charging of the zeolite was effected with a charging solution containing 800 mg of sodium salt of ketoprofene dissolved in 30 ml of ethyl alcohol.

For the preparation of all the compositions, the dispersions containing the zeolites were kept under stirring for 48 hours, at room temperature. At the end of the charging, the solutions were filtered. After filtering, the zeolites charged with ketoprofene were subsequently treated, under vacuum, in a rotating evaporator (Rotavapor) to eliminate the residual solvent.

The quantity of ketoprofene present in each pharmaceutical composition i.e. charged onto the release medium, was determined gravimetrically. The gravimetric determination was repeated three times on each sample and the standard deviation of the results obtained was calculated. The results of the gravimetric determination are indicated in Table 3.

**Table 3. Charging of ketoprofene**

| **Composition nr.** | **Sample** | **Activation** | **Charging percentage Ketoprofene*** | **Charging percentage Ketoprofene sodium salt**** |
|---|---|---|---|---|
| 1 | A | Yes | 28.5 ± 1.1 | -- |
| 2 | B | Yes | 28.5 ± 1.2 | -- |
| 3 | A | No | 6.9 ± 2.2 | -- |
| 4 | B | No | 8.79 ± 1.3 | -- |
| 5 | A | Yes | -- | 2.0 ± 0.7 |
| 6 | B | Yes | -- | 2.4 ± 0.5 |
| 7 | B | No | -- | 0.5 ± 0.02 |

| | | | | |
|---|---|---|---|---|
| * Weight percentage of ketoprofene with respect to the total weight of the charged zeolite. ** Weight percentage of sodium salt of ketoprofene with respect to the total weight of the charged zeolite. | | | | |

In the case of zeolites X and ALSX activated and charged with solutions of ketoprofene in ethyl ether (compositions 1 and 2), the gravimetric data indicate that all the 800 mg of ketoprofene were charged onto both the zeolites at the end of the impregnation process.

The values indicated in the table refer in fact to the zeolites charged, by expressing these values in examples 1 and 2 with respect to the zeolites activated, a percentage equal to 39.9% is obtained, consequently on 2 grams of zeolite all the 800 mg of drug, corresponding to 40% by weight, were effectively charged.

The charging levels of the zeolites which were reached using solutions of sodium salts of ketoprofene in ethyl alcohol, on the contrary, were low (less than 2.5% by weight with respect to the total weight of the zeolite).

For both zeolites, the gravimetric data show that the charging of the ketoprofene molecules takes place to a negligible degree on the non-activated zeolites, regardless of the type of solution used for the charging. This shows that the removal of the water molecules from the pores of the zeolite is essential for guaranteeing the subsequent charging of the pharmacologically active molecules.

### Example 3 - Characterization of the compositions

The presence of ketoprofene molecules in the pharmaceutical compositions and in particular in the release means was confirmed by X-ray diffractometry (XRD).

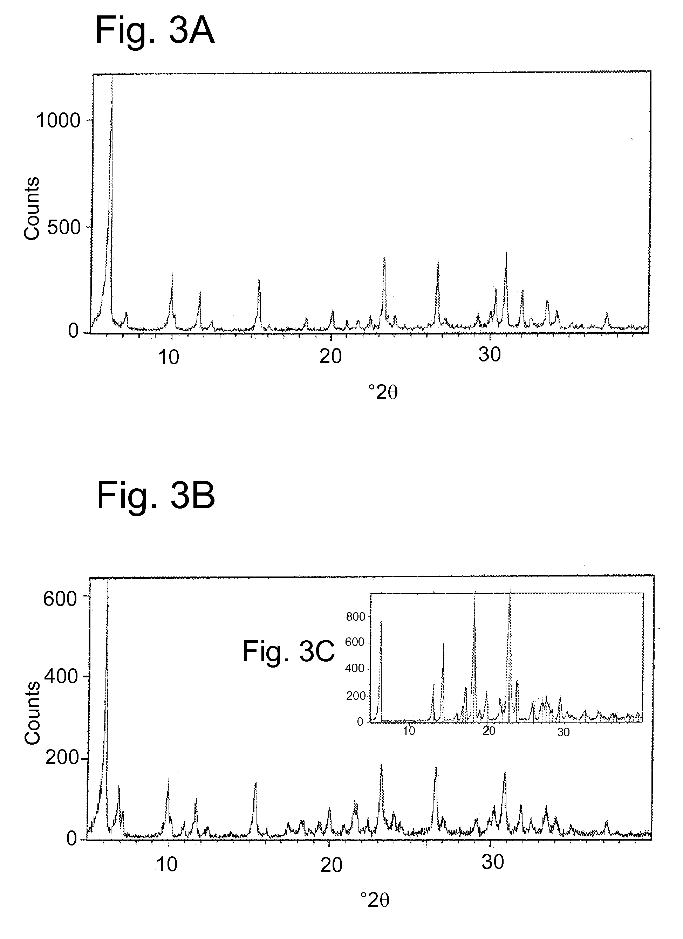

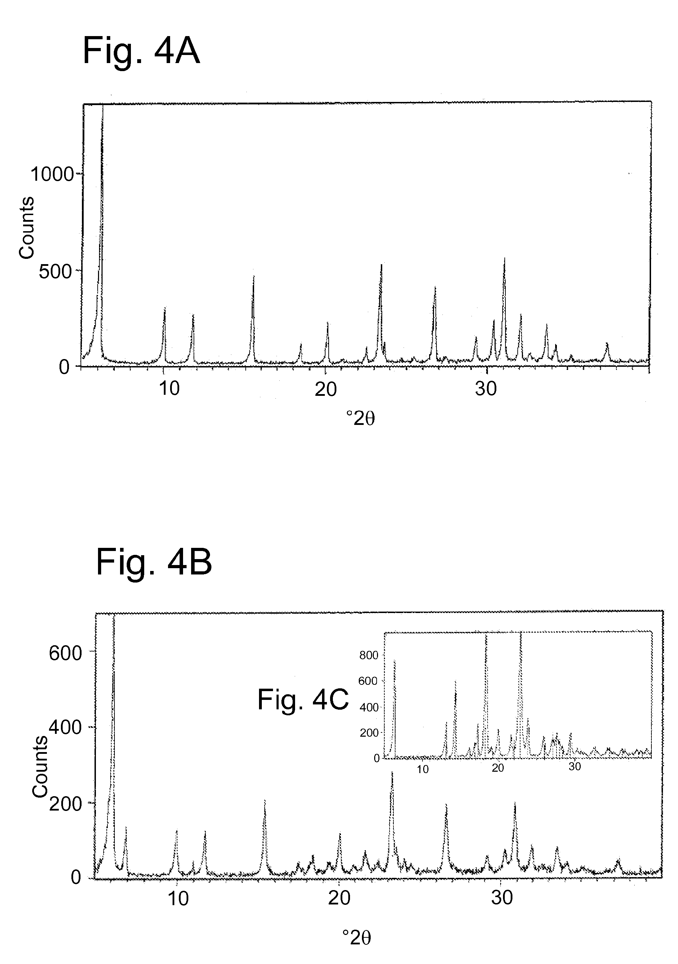

Figures 3a and 3b show the XRD spectra of zeolite ALSX respectively before (Sample B activated) and after charging with ketoprofene (composition Nr. 2). Figures 4a and 4b show the XRD spectra of Zeolite X, respectively before (Sample A activated) and after charging with ketoprofene (composition Nr. 1).

From a comparison of the spectra of the two zeolites, before and after charging, with that of ketoprofene (indicated in the square of Figure 3c and Figure 4c), it is possible to distinguish, within the range of 15-25 2θ of the spectra relating to compositions 1 and 2, the signals associated with ketoprofene confirming the incorporation of ketoprofene in the zeolite.

More specific information on the localization of the ketoprofene molecules on the release medium in the pharmaceutical compositions were obtained from the adsorption measurements of gaseous N₂ in compositions 1 and 2. These measurements provide quantitative information about the surface area and pore volume of the zeolites. The measurements were effected with a Quantachrome Autosorb 1-MP instrument on the starting zeolites (Samples A and B) not activated and not charged, after treatment of the samples at 300°C for 16 hours before the adsorption measurements of N₂. The activated and charged zeolites (compositions 1 and 2), on the other hand, were subjected to heating treatment at 60°C before the adsorption of N₂ to avoid desorption of the ketoprofene molecules (the melting point of ketoprofene is about 94°C).

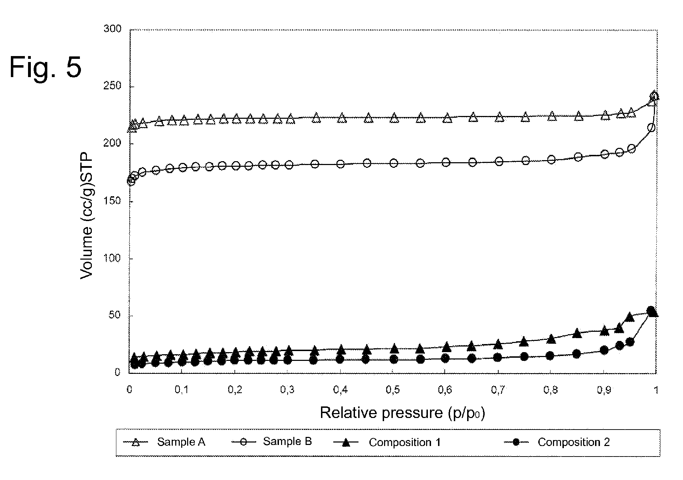

Figure 5 shows the N₂ adsorption isotherms obtained for the zeolites before (Samples A and B) and after charging (compositions 1 and 2). The decrease in the surface accessible to gaseous nitrogen together with the reduction in the volume accessible of the pores (Table 4) show that the ketoprofene molecules are situated both inside the pores and on the surface of both zeolites.

**Table 4. N₂ adsorption measurements**

| **Sample** | **Surface (BET) m²g⁻¹** | **Pore volume Cm³g⁻¹** |
|---|---|---|
| A. | 660 | 0.377 |
| Composition 1* | 60.3 | 0.083 |
| B. | 543 | 0.374 |
| Composition 2* | 36.4 | 0.077 |

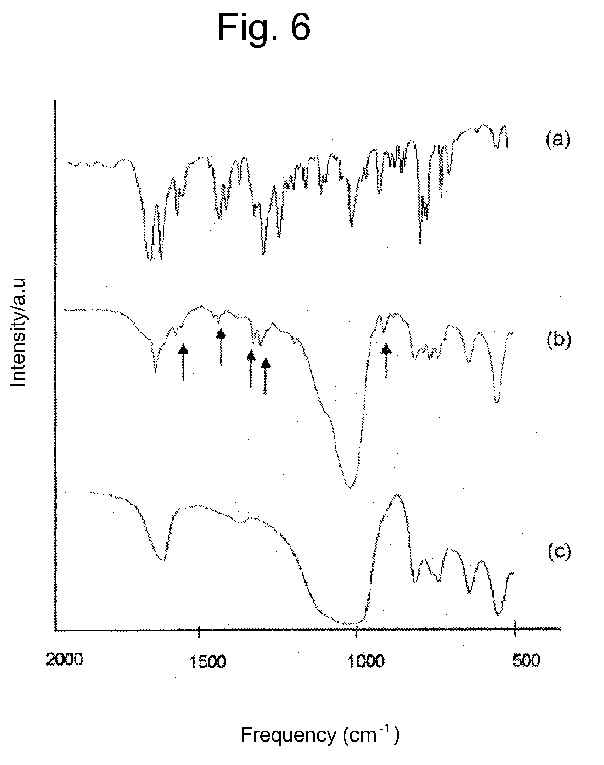

Further evidence that the ketoprofene molecules are incorporated in the pores of zeolite ALSX were obtained from comparing the FTIR spectrum of ketoprofene with the spectra of the ALSX zeolites before (Sample B) and after charging (composition 2). The spectra were recorded with a Thermofinnigan NICOLET 380 spectrometer and are indicated in Figure 6. The arrows in Figure 6b indicate the absorption peaks of the IR radiations characteristic of ketoprofene molecules which are visible in the spectrum of composition 2 (Figure 6, spectrum b). The spectra of zeolite X, before (Sample A) and after charging of ketoprofene (composition 1), are very similar to those shown in figure 6 with reference to zeolite ALSX and for this reason are not indicated.

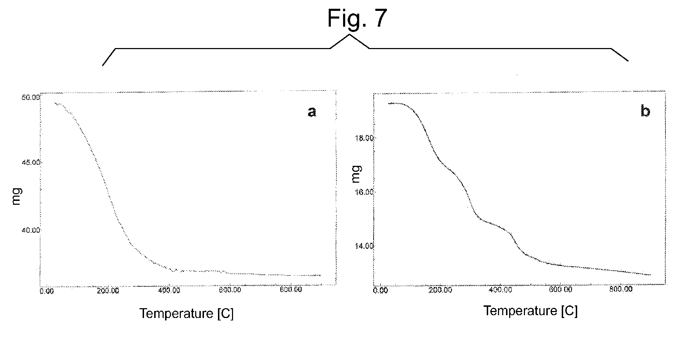

The zeolites charged with ketoprofene (compositions 1 and 2) were also subjected to thermogravimetric measurements (Shimadzu DTG60 instrument) effected within a temperature range of 25 to 900°C and with a heating rate of the sample of 20°C min⁻¹. The thermogravimetric data (Table 5) confirm the complete charging of ketoprofene in the zeolites. The thermogravimetric plots of zeolite ALSX before (Sample B) and after charging (composition 2) are indicated in Figure 7.

The greatest weight loss by heating is observed between 25°C and 430°C, probably corresponding to a combined effect of evaporation of the residual solvent, degradation of the molecules of the drug and desorption of the fraction of water molecules and ketoprofene adsorbed on the surface. The remaining ketoprofene is totally removed from the composition within the temperature range of 430°C - 900°C. The weight loss within this temperature range indicates that the molecules of the drug are quite strongly adsorbed on the surface of the zeolite.

The thermogravimetric behaviour of zeolite X before (Sample A) and after charging with ketoprofene (composition 1) is very similar to that observed for zeolite ALSX (not shown).

**Table 5. - Thermogravimetric analysis (weight loss percentages referring to the total weight of the zeolite charged with ketoprofene).**

| | **25-430** | **431-513** | **514-900** | **25-900** |
|---|---|---|---|---|
| | **(°C)** | **(°C)** | **(°C)** | **(°C)** |
| Composition 1 | -25.14% | -4.6% | -3.7% | -33.4% |
| Composition 2 | -25.14% | -4.8% | -3.3% | -33.2% |

### Example 4 - Release kinetics of ketoprofene

The studies on the release kinetics of ketoprofene were effected by means of tests in vitro using the following procedure: 10 mg of zeolite charged with the drug (compositions 1 and 2) were dispersed in a solution of HCl 0.1 N (pH 1.0 - to simulate conditions of gastric acidity). The dispersion was kept under continuous stirring, at 37°C for 90 minutes. 26.7 ml of a buffer solution consisting of an aqueous solution of Na₂HPO₄ (0.2 M) and NaOH (0.1 N), were subsequently added to the dispersion, which raised the pH to a value of 5.0. Finally, after a further 60 minutes, a further 15.8 ml of the above buffer solution were added to the dispersion until a pH = 6.8 was reached. These conditions of pH and ionic composition of the solution containing the zeolite-based pharmaceutical composition simulate conditions of the gastro-intestinal tract in which a pharmaceutical composition administered orally can be found.

In order to quantitatively evaluate the release degree of ketoprofene molecules under the various acidity conditions and in relation to the time, an aliquot of the dispersion containing the composition was taken every 15 minutes, filtered and injected into an HPLC chromatograph having the following technical characteristics:
- instrument : Shimadzu HPLC
- spectrophotometric detector: UV SPD-10AVvp,
- two LC-10ADvp pumps with a low-pressure gradient system,
- injection loop: Rheodyne Model 7725i (volume = 20 µl) ,
- Phenomenex C18 analytical column (150 x 4.60 mm; 5 µm) ,
- Mobile phase: mixture, in a 50:50 ratio, of acetonitrile and an aqueous solution of acetic acid (5 g/l) (flow = 1 ml/min).

The elution was monitored with the UV detector at a wave-length of 260 nm. Each chromatographic passage had a duration of 10 minutes. Under the above operating conditions, the ketoprofene showed a retention time of 5.4 minutes. The calibration curve was constructed using, as external standards, 4 solutions at a concentration of ketoprofene within the range of 10-50 µM, plus a blank.

The above tests in vitro revealed the release capacity of the compositions according to the present invention based on release means of zeolites charged with ketoprofene.

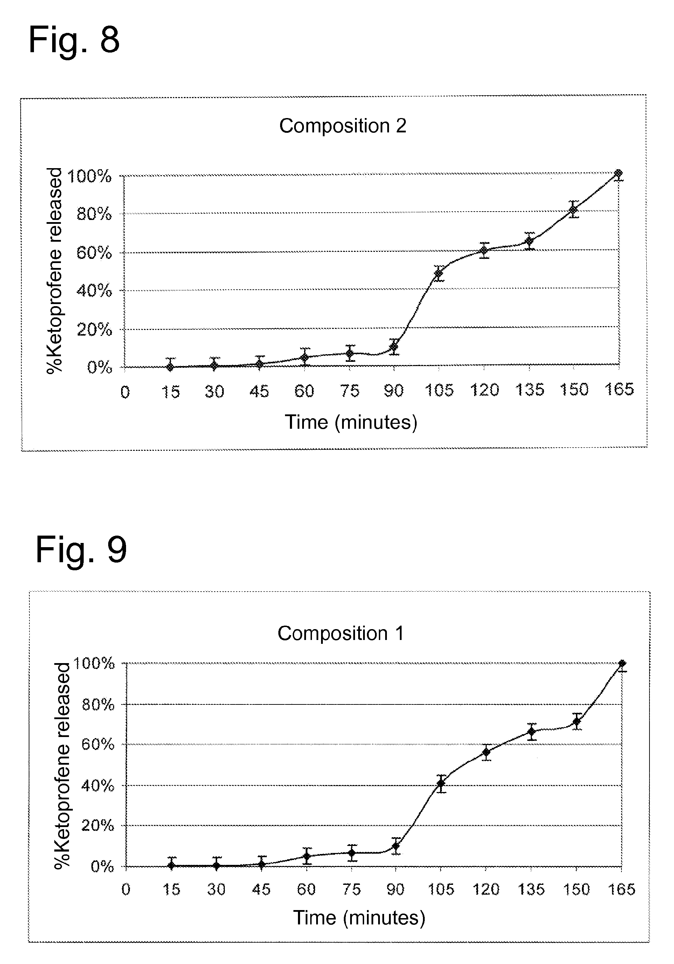

Figure 8 and Figure 9 show the graphs relating to the dependence of the released quantity of ketoprofene in relation to the time, under the in vitro test conditions, for zeolite X (composition 1) and for zeolite ALSX (composition 2).

It was observed that during the first 90 minutes, when the pH of the solution is equal to 1.0, the overall release of ketoprofene from both compositions is equal to about 10% by weight of the overall quantity charged on the zeolite.

With the subsequent addition of the buffer solution, which causes a rise in the pH to 5.0, the quantity of pharmacologically active molecule which is released, progressively increases with time up to about 80% in the case of composition 2.

The completion of the release of ketoprofene is observed only with the subsequent addition of buffer solution, i.e. when a pH value of about 6.8 is reached.

## Claims

1. A pharmaceutical composition for the oral administration of pharmacologically active molecules, comprising a release means consisting of porous zeolite in powder form incorporating pharmacologically active molecules.

2. The composition according to claim 1, wherein the porous zeolite in powder form incorporates pharmacologically active molecules inside the pores and/or on its surface.

3. The composition according to claim 1 or 2, wherein the porous zeolite is selected from zeolites belonging to the group of faujasites (type Y, X, ALSX) or to the group of A-type zeolites (LTA), even more preferably X-type zeolites and/or ALSX.

4. The composition according to any of the claims from 1 to 3, wherein the pharmacologically active molecules are molecules of a non-steroidal anti-inflammatory drug (FANS).

5. The composition according to any of the claims from 1 to 4, wherein the pharmacologically active molecules are molecules selected from the group comprising indomethacin, diclofenac, ketorolac, ibuprofene, ketoprofene, naproxen, flurbiprofene, mefenamic acid, meclofenamate, rofecoxib, celecoxib, etoricoxib, pyroxicam, meloxicam, tenoxicam, lornoxicam, cinnoxicam, nimesulide and/or mixtures thereof, preferably molecules of ketoprofene.

6. The composition according to any of the claims from 1 to 5, wherein the release means consists of porous zeolite in powder form of X- or ALSX- type and the pharmacologically active molecules are ketoprofene.

7. A process for the preparation of a pharmaceutical composition for the oral administration of pharmacologically active molecules, comprising a release means consisting of porous zeolite in powder form, incorporating pharmacologically active molecules, according to any of the claims from 1 to 6, said process comprising the following operational phases:
a) heating the porous zeolite in powder form to a temperature within the range of 200 to 600°C, for a time varying from 0.5 to 16 hours;
b) putting the zeolite obtained from phase a) in contact, at a temperature ranging from 25 to 30°C for a time ranging from 36 to 48 hours, with a charging solution comprising pharmacologically active molecules dissolved in a solvent or a mixture of solvents;
c) separating the zeolite from the solvent.

8. The process according to claim 7, wherein phase a) is carried out at a temperature within the range of 300 to 400°C for a time varying from 3 to 4 hours.

9. The process according to any of the claims from 7 to 8, wherein the solvent of the charging solution is selected from ethyl ether, ethyl alcohol, acetone and/or mixtures thereof, preferably ethyl ether or ethyl alcohol.

10. The process according to any of the claims from 7 to 9, wherein the concentration of the pharmacologically active molecules in the charging solution varies from 10 g/l to 50 g/l, preferably from 20 g/l to 30 g/l.

11. The process according to any of the claims from 7 to 10, wherein phase c) comprises the separation of the zeolite from the solvent by filtration and the subsequent treatment of the zeolite thus separated in an evaporator under vacuum.

12. Release means for the oral administration of pharmacologically active molecules, comprising a porous zeolite in powder form selected from zeolites belonging to the group of faujasites (type Y, X, ALSX) or to the group of A-type zeolites (LTA), even more preferably X-type zeolites and/or ALSX.

13. Use of the pharmaceutical composition comprising a release means consisting of a porous zeolite in powder form, incorporating pharmacologically active molecules, for the preparation of drugs for oral administration, in particular for the treatment of inflammatory pathologies at an intestinal level.

14. Use of the pharmaceutical composition according to any of the claims from 1 to 6 for releasing pharmacologically active molecules in the intestine of mammals.

15. Use of the pharmaceutical composition according to any of the claims from 1 to 6 for the preparation of a pharmaceutical composition for the treatment of inflammatory pathologies in mammals.

16. Use of the pharmaceutical composition according to any of the claims from 1 to 6 for the preparation of a pharmaceutical composition for the treatment of ulcerative colitis.

17. Use of the pharmaceutical composition according to any of the claims from 1 to 6 for the preparation of a pharmaceutical composition for the treatment of Crohn's disease.
